Europäisches Patentamt

European Patent Office

Office européen des brevets

(11) Veröffentlichungsnummer: **0 314 880 B1**

(12) # EUROPÄISCHE PATENTSCHRIFT

(45) Veröffentlichungstag der Patentschrift: **30.09.92**

(51) Int. Cl.⁵: **A61M 5/14**

(21) Anmeldenummer: **88111732.9**

(22) Anmeldetag: **21.07.88**

(54) **Druckinfusionsapparat.**

(30) Priorität: **04.11.87 DE 3737331**

(43) Veröffentlichungstag der Anmeldung:
**10.05.89 Patentblatt 89/19**

(45) Bekanntmachung des Hinweises auf die
Patenterteilung:
**30.09.92 Patentblatt 92/40**

(84) Benannte Vertragsstaaten:
**AT CH DE ES FR GB IT LI NL SE**

(56) Entgegenhaltungen:
EP-A- 0 142 736      EP-A- 0 164 904
DE-B- 2 724 538      DE-U- 8 137 235
GB-A- 2 115 495      US-A- 4 424 720
US-A- 4 627 835

(73) Patentinhaber: **B. Braun Melsungen AG**
**Carl-Braun Strasse**
**W-3508 Melsungen(DE)**

(72) Erfinder: **Mardorf, Robert**
**Zur Schlade 29**
**W-3508 Melsungen(DE)**
Erfinder: **Pröger, Wolfgang**
**Steinweg 13**
**W-3509 Spangenberg-Elbersdorf(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

**Beschreibung**

Die Erfindung betrifft einen Druckinfusionsapparat nach dem Oberbegriff des Patentanspruchs 1.

Ein bekannter Druckinfusionsapparat, nach DE-U-81 37 235 weist ein Gehäuse und einen relativ zu dem Gehäuse durch einen Motorantrieb verschiebbaren Schieber auf. An dem Gehäuse ist ein erster Spritzenhalter angebracht, an dem der Zylinder einer Spritze befestigt werden kann, und an dem Schieber ist ein zweiter Spritzenhalter angebracht, an dem das rückwärtige Ende der Kolbenstange der Spritze befestigt werden kann. Wird der Schieber über einen Spindelantrieb angetrieben, dann bewegt sich der zweite Spritzenhalter in Richtung auf den ersten Spritzenhalter, wodurch das in der Spritze befindliche Medikament ausgedrückt wird. Für Endabschaltungen und Notabschaltungen ist ein Drucksensor vorgesehen, der mit dem ersten Spritzenhalter gekoppelt ist. Der erste Spritzenhalter kann gegen die Kraft einer Feder relativ zu dem Gehäuse bewegt werden, wenn der Kolben im Spritzenzylinder seine vordere Endstellung erreicht hat oder wenn durch Abknicken der aus dem Spritzenzylinder herausführenden Leitung ein Flüssigkeitsstau im Spritzenzylinder entsteht. Durch die Bewegung des ersten Spritzenhalters wird ein Schalter betätigt, der eine Alarmauslösung und eine Stillsetzung des Antriebs bewirkt. Der Schalter ist gehäuseseitig vorgesehen und eine Feder, die den ersten Spritzenhalter in die Ruhestellung zieht, ist ebenfalls am Gehäuse des Druckinfusionsapparates verankert. Das Tastelement in Form des elektromechanischen Schalters ist mit seitlichem Abstand von der Spritzenachse angeordnet. Dadurch entstehen zusätzliche Biege-oder Reibmomente bei der Kraftübertragung von dem ersten Spritzenhalter auf den gehäuseseitig montierten Drucksensor, mit der Folge, daß die Druckabschaltwerte streuen und nicht mit der erforderlichen Zuverlässigkeit eingehalten werden können.

Ein weiterer Nachteil der bekannten Druckinfusionsapparate besteht darin, daß das ordnungsgemäße Einlegen einer Spritze nicht überwacht wird. Durch fehlerhaftes Einlegen der Spritze kann es vorkommen, daß der Druckinfusionsapparat arbeitet, ohne daß dem Patienten Flüssigkeit infundiert wird. Bei mobilen Druckinfusionsapparaten besteht ein zusätzlicher Störfaktor darin, daß beim Tragen des Apparates gegen ein Hindernis gestoßen werden kann, wobei die Spritze aus ihrer Halterung springen kann, ohne daß dies erkannt wird. Die Folge ist, daß bei anschließendem Betrieb des Druckinfusionsapparates entweder keine Infusion durchgeführt wird oder der Apparat aufgrund des hydrostatischen Drucks in der Infusionsleitung innerhalb kurzer Zeit entleert wird.

Ein Druckinfusionsapparat, von dem der Oberbegriff des Patentanspruchs 1 ausgeht, ist bekannt aus US-A- 4 627 835. Dieser Druckinfusionsapparat erfordert das Anbringen eines Konnektors an dem vorderen Ende des Spritzenzylinders. Dieser Konnektor stützt sich an dem festen Spritzenhalter des Druckinfusionsapparates ab und er ist so ausgebildet, daß er zwei Abstützflächen aufweist, von denen jede wahlweise an den festen Spritzenhalter angesetzt werden kann, damit Spritzen unterschiedlicher Abmessungen verwendet werden können. An dem festen Spritzenhalter ist ein Drucksensor vorgesehen, an den die jeweils benutzte Abstützfläche des Konnektors angesetzt werden kann und der eine federgespannte schwenkbare Platte aufweist. Wenn der Druck im Spritzenzylinder beispielsweise infolge des Abknickens der angeschlossenen Schlauchleitung zunimmt, betätigt der Konnektor den Drucksensor, der daraufhin einen Schaltvorgang ausführt und Alarm erzeugt oder den Druckinfusionsapparat abschaltet.

Bei dem bekannten Druckinfusionsapparat ist ferner in Höhe des Konnektors ein Spritzendetektor angeordnet, der den Durchmesser des in seiner Nähe befindlichen Konnektorteils erkennt und die betreffende Position des Konnektors einem Mikroprozessor mitteilt. Auf diese Weise erhält der Mikroprozessor eine Information darüber, in welcher der beiden möglichen Stellungen der Konnektor an den Spritzenhalter angesetzt wurde, und gegebenenfalls auch eine Information darüber, ob überhaupt ein Konnektor vorhanden ist. Das Vorhandensein einer Spritze im Druckinfusionsapparat wird nicht unmittelbar erkannt.

Aus EP-A-0 164 904 A2 ist ein Druckinfusionsapparat bekannt, bei dem der bewegbare Spritzenhalter einen Spritzendetektor enthält, der einen durch einen Kipphebel betätigten Schalter aufweist. Beim Einsetzen des Kolbenstangenendes in den bewegbaren Spritzenhalter wird der Spritzendetektor betätigt.

Bei einem in US-A-4 424 720 beschriebenen Druckinfusionsapparat besteht der bewegbare Spritzenhalter aus einem Schwenkarm, der seitlich über einen Flansch an der Kolbenstange der Spritze geschwenkt wird und an einem drehbaren Schaft befestigt ist. Wenn der Schwenkarm diejenige Schwenkstellung erreicht hat, die der Betriebsstellung entspricht, betätigt ein am Schaft befestigter Nocken einen elektro-optischen Schalter, wodurch angezeigt wird, daß der Spritzenhalter sich in der Betriebsstellung befindet. Voraussetzung hierfür ist nicht, daß eine Spritze in den Druckinfusionsapparat eingelegt ist. Wenn der Schwenkarm in die Betriebsstellung gebracht wurde und sich dabei zunächst außerhalb des Bereiches der Kolbenstange befindet, kann er vorwärtsgeschoben werden, bis er an dem Flansch der Kolbenstange angreift. Ein Drucksensor oder ein Spritzendetektor ist hierbei nicht vorgesehen.

Druckinfusionsapparate haben häufig ein Betätigungsorgan, das eine manuelle Nachstellung des Schiebers zum Belüften der Spritze und der Infusionsleitung und ein manuelles Anstellen des Schiebers auf die jeweilige Spritzenlänge ermöglicht. Erfolgt während des Betriebs des Apparates eine Verstellung des Schiebers durch Betätigen des Betätigungsorgans, um einen zusätzlichen Medikamentenbolus zu geben oder auch versehentlich, so wird dies vom Steuersystem des Druckinfusionsapparats nicht erkannt.

Der Erfindung liegt die Aufgabe zugrunde, einen Druckinfusionsapparat zu schaffen, bei dem der Drucksensor und der Spritzendetektor in demselben Spritzenhalter vereinigt sind.

Die Lösung dieser Aufgabe erfolgt erfindungsgemäß mit den im Patentanspruch 1 angegebenen Merkmalen.

Mit der Erfindung wird erreicht, daß der Druckinfusionsapparat nur dann betrieben werden kann, wenn eine Spritze ordnungsgemäß in die beiden Spritzenhalter eingesetzt ist. Der Spritzendetektor erkennt das lagerichtige Einsetzen der Spritze in den betreffenden Spritzenhalter und erzeugt ein Signal zum Inbetriebsetzen des Antriebs nur dann, wenn eine Spritze so eingesetzt ist, daß sie in beiden Spritzenhaltern abgestützt ist.

Eine weitere Aufgabe der Erfindung besteht darin, einen Druckinfusionsapparat zu schaffen, bei dem manuelle Verstellungen des Schiebers erkannt werden. Dies geschieht mit den Merkmalen des Patentanspruchs 4. Der Ausrastdetektor erkennt, ob das Betätigungsorgan ausgerastet ist, um Verstellungen des Schiebers zu ermöglichen. Ist das Betätigungsorgan ausgerastet, so schaltet der Ausrastdetektor den Antrieb ab. Ferner kann ein Alarmsignal erzeugt werden, das der Bedienungsperson angibt, daß ein manueller Eingriff in den Betrieb des Druckinfusionsapparates erfolgt.

Gemäß einer bevorzugten Weiterbildung der Erfindung wird für den Drucksensor, den Spritzendetektor und ggf. auch noch für den Ausrastdetektor nur ein einziger Schalter benutzt. Dieser Schalter kann ein berührungsloser Schalter, z.B. eine Lichtschranke oder ein induktiver Näherungsschalter, sein. Durch Verwendung eines einzigen Schalters, der durch mehrere Überwachungselemente ausgelöst werden kann, werden der apparative Aufwand und die Größe des Druckinfusionsapparates verringert.

Im folgenden wird unter Bezugnahme auf die Zeichnungen ein Ausführungsbeispiel der Erfindung näher erläutert.

Es zeigen:

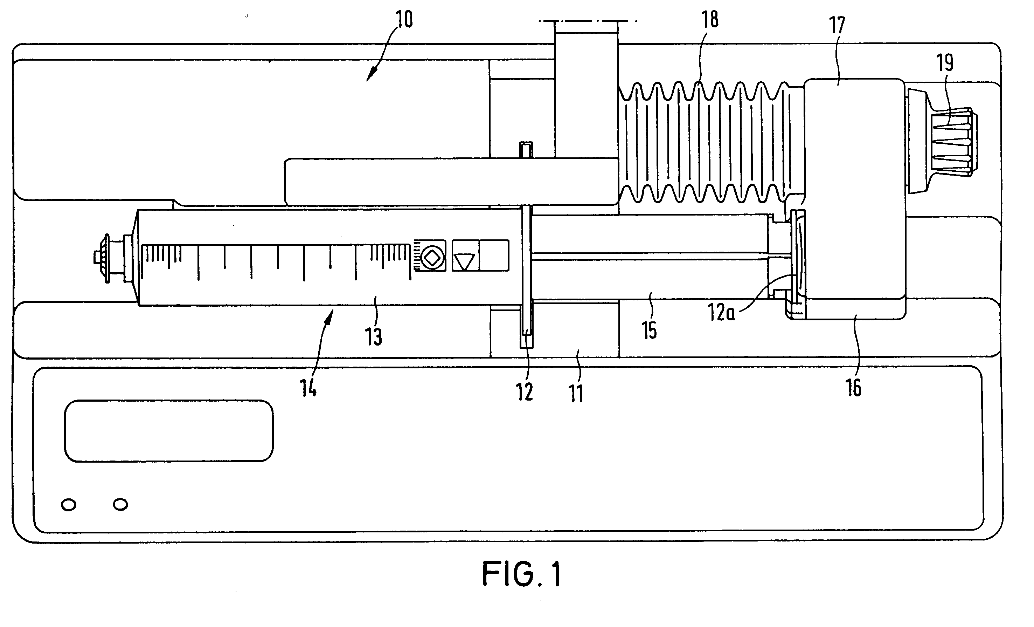

Fig. 1    eine schematische Draufsicht des Druckinfusionsapparates,

Fig. 2    einen Horizontalschnitt durch den Druckinfusionsapparat,

Fig. 3    eine Explosionsdarstellung des verwendeten Schalters in Form einer Lichtschranke,

Fig. 4    einen Teil-Längsschnitt des Schiebers und des zweiten Spritzenhalters in der Betriebsstellung,

Fig. 5    einen Längsschnitt gemäß Fig. 4, bei nicht eingesetzter Spritze,

Fig. 6    einen Längsschnitt gemäß Fig. 4, bei entriegeltem Betätigungsorgan, und

Fig. 7    einen Längsschnitt gemäß Fig. 4 bei Druckabschaltung infolge eines zu hohen Kolbenwiderstandes der Spritze.

Der Druckinfusionsapparat weist ein Gerätegehäuse 10 auf, an dem ein erster Spritzenhalter 11 fest angebracht ist. In den Spritzenhalter 11 wird der Flansch 12 des Zylinders 13 einer Spritze 14 eingesetzt. Die Kolbenstange 15 der Spritze 14 weist am Ende einen Flansch 12a auf, der in den zweiten Spritzenhalter 16 eingesetzt wird, welcher an einem Schieber 17 befestigt ist, der relativ zum Gehäuse 10 linear verschiebbar ist. Zwischen dem Schieber 17 und dem Gehäuse 10 erstreckt sich ein Faltenbalg 18, der den Spindeltrieb zum Bewegen des Schiebers 17 umschließt. Am Schieber 17 ist ein Betätigungsorgan 19 in Form eines ausrastbaren Drehknopfes angebracht, um den Schieber zusammen mit dem Spritzenhalter 16 manuell bewegen zu können.

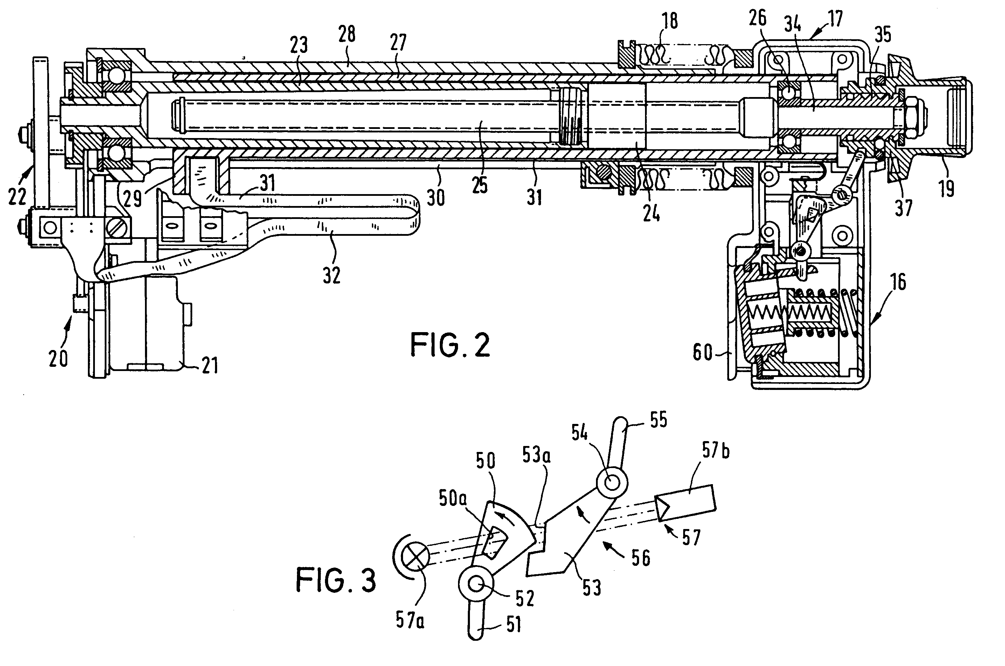

Der Antrieb 20 für die Bewegung des Schiebers 17 weist einen am Antriebsgehäuse 28 befestigten Elektromotor 21 auf, dessen Drehkraft über ein Zahnradgetriebe 22 auf ein drehbar im Antriebsgehäuse 28 gelagertes Rohr 23 übertragen wird (Fig. 2). Das Rohr 23 erstreckt sich in Richtung auf den Schieber 17 und an seinem schieberseitigen Ende ist eine Spindelmutter 24 befestigt, die über ein (nicht dargestelltes) Kugelumlaufgetriebe mit der Spindel 25 in Eingriff steht. Die Spindel 25 ist eine Kugelgewindespindel. Durch das Zusammenwirken des in der Spindelmutter 24 enthaltenen Kugelumlaufgetriebes mit der Kugelgewindespindel wird erreicht, daß der Spindeltrieb nahezu reibungsfrei arbeitet und nicht-selbsthemmend ist.

Das Herausziehen des Schiebers 17 erfolgt manuell bei ausgerastetem Betätigungsorgan 19. Das Führungsrohr 27 enthält einen (nicht dargestellten) Führungsrohreinsatz aus Kunststoff, in dem das Ende der Spindel 25 im Schieber 17 gelagert ist. Das Führungsrohr 27 ist am Schieber befestigt und erstreckt sich über das Rohr 23. Das Führungsrohr 27 wird von dem rohrförmigen Antriebsgehäuse 28 umschlossen. An dem äußeren Ende des Antriebsgehäuses 28 ist das eine Ende des Faltenbalges 18 befestigt. Das andere Ende des Faltenbalges ist an dem Schieber 17 befestigt.

Wenn der Antrieb 20 das Rohr 23 zusammen mit der Spindelmutter 24 dreht, wird dadurch die Spindel 25 axial verschoben und das Führungsrohr 27 wird teleskopisch in das Antriebsgehäuse 28 eingefahren, wodurch der Schieber 17 zusammen mit dem Spritzenhalter 16 bewegt wird. Eine Verdrehung des Schiebers 17 wird dadurch verhindert, daß am gehäuseseitigen Ende des Führungsrohreinsatzes ein radial abstehender Ansatz 29 vorgesehen ist, der durch einen Längsschlitz 30 des Rohres 28 nach außen vorsteht. Durch den Ansatz 29 hindurch führt ein flexibles Kabel 31, das außerhalb des Führungsrohrs 27 eine Kabelschleife 32 bildet, welche in das Gehäuse 10 hineinführt. Das Kabel 31 ist mit einer Leitung verbunden, die sich vom Ansatz 29 längs des Führungsrohres 27 bis in den Schieber 17 erstreckt.

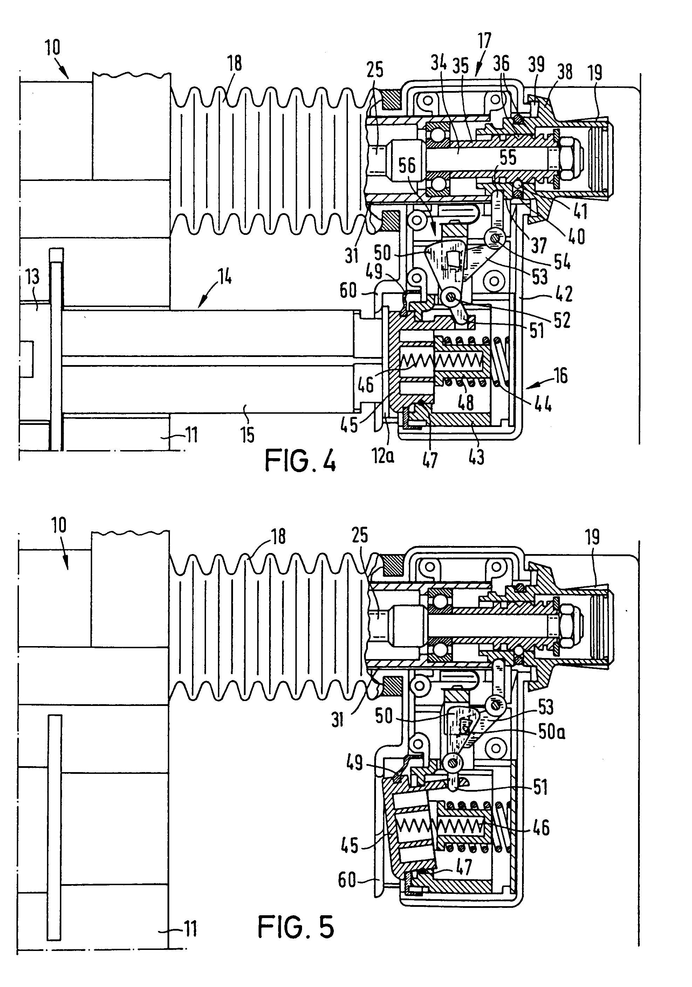

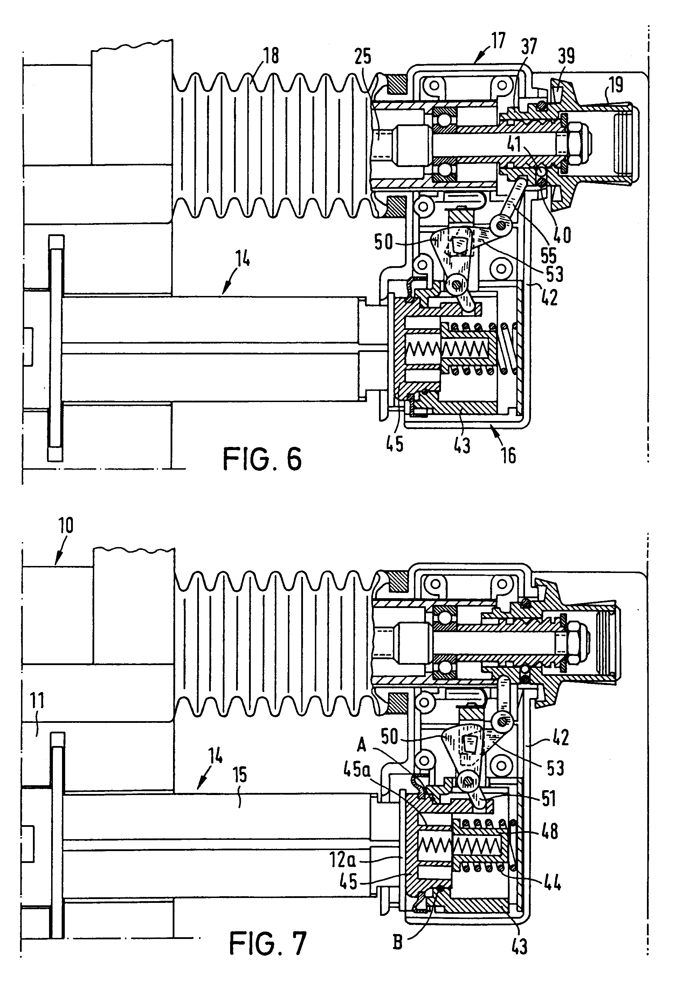

Das schieberseitige Ende der Spindel 25 ist als Schaft 34 ausgebildet, auf dem eine mit Ringnuten 36 versehene Hülse 35 sitzt (Fig. 4). Auf der Hülse 35 sitzt die Hülse 37 des Betätigungsorgans 19. Dieses Betätigungsorgan ist als Drehknopf ausgebildet, der einen seitlich abstehenden Flansch 38 mit stirnseitiger Verzahnung 39 aufweist, welche mit Rastausnehmungen des Schiebers 17 zusammengreift. Die Hülse 37 des Betätigungsorgans 19 wird durch einen Spannring 40 radial zusammengedrückt und gegen die Hülse 35 gepreßt. Der Spannring 40 drückt drei Kugeln 41 in eine der Ringnuten 36, um eine Raste zu bilden, die das Betätigungsorgan 19 relativ zu der Hülse 35 in axialer Richtung fixiert. Durch Ziehen an dem Betätigungsorgan 19 kann dieses relativ zu der Hülse 35 und zur Spindel 25 verschoben werden (Fig. 6), wobei die Kugel 41 von einer Ringnut 36 in die andere Ringnut 36 springt. Dabei gelangt die axiale Verzahnung 39 außer Eingriff mit dem Gehäuse des Schiebers 17 und das Betätigungsorgan 19 kann gedreht werden, um die Spindel 25 manuell zu drehen. Der zweite Spritzenhalter 16 ist fest mit dem Schieber 17 verbunden, wobei das Gehäuse 42 des Schiebers zugleich das Gehäuse des Spritzenhalters 16 bildet. In diesem Gehäuse ist ein hülsenförmiges Tastelement 43 untergebracht, das längs der Achse einer in den Druckinfusionsapparat eingesetzten Spritze 14 verschiebbar ist. Das Tastelement 43 wird von einer am Schiebergehäuse 42 abgestützten Feder 44 in Richtung auf den ersten Spritzenhalter 11 gedrückt und stößt dabei gegen einen Anschlag. An dem dem Spritzenhalter 11 zugewandten Ende des Tastelementes 43 befindet sich eine Tastkappe 45, die von einer zweiten Feder 46 in Richtung auf den Spritzenhalter 11 gedrückt wird. Die Tastkappe 45 ist in der vorderen Öffnung des Tastelements 43 geführt und an einer Stelle ihres Umfangs über einen Stift 47 gelenkig mit dem Rastelement 43 verbunden, so daß sie sich im entlasteten Zustand gemäß Fig. 5 unter dem Druck der Feder 46 schrägstellt. Die Feder 46 ist in einem Becher 48 abgestützt, gegen den die Feder 44 drückt. Die Feder 46 ist wesentlich weicher als die Feder 44. Zwischen dem Umfangsrand der Tastkappe 45 und dem Gehäuse 42 erstreckt sich eine Membran 49 zur Abdichtung des Gehäuseinneren. Vor der Tastkappe 45 ist am Gehäuse 42 eine Gabel 60 vorgesehen, in die das Ende der Kolbenstange 15 so eingesteckt werden kann, daß der Flansch 12a der Kolbenstange hinter die Zinken der Gabel 60 greift, während das stirnseitige Ende des Flansches sich flach gegen die Tastkappe 45 legt und diese um die Achse 47 herum geradestellt.

In eine seitliche Ausnehmung der Tastkappe 45 greift ein Stift 51 der Schaltfahne 50 ein, die um eine Achse 52 des Tastelements 43 schwenkbar angebracht ist. Eine weitere Schaltfahne 53, die an einem Rand eine Ausnehmung 53a aufweist, ist parallel zur Schaltfahne 50 angeordnet. Die Schaltfahne 53 ist um eine Achse 54 des Gehäuses 42 schwenkbar und sie wird von einem Stift 55 gesteuert, der in eine Umfangsnut der Hülse 37 des Betätigungsorgans 19 eingreift.

Wie Fig. 3 zeigt, bilden die Schaltfahnen 50 und 53 zusammen mit einer Lichtschranke einen berührungslosen Schalter 56. Die am Tastelement 43 befestigte Lichtschranke 57 besteht aus dem Lichtsender 57a und dem fotoelektrischen Empfänger 57b. Im Lichtweg vom Sender 57a zum Empfänger 57b sind die Schaltfahnen 50 und 53, die quer zu diesem Lichtweg verschwenkbar sind, angeordnet. Die Schaltfahne 50 weist eine Öffnung 50a auf, und die Schaltfahne 53 eine Ausnehmung 53a. Wenn sich beide Schaltfahnen 50 und 53 in der Betriebsstellung befinden, geht der Lichtweg durch die Öffnung 50a und die Ausnehmung 53a hindurch, so daß das Licht auf den Empfänger 57b fällt. In diesem Fall erzeugt der Schalter 57 ein elektrisches Signal, das über die Leitung 31 zu einer (nicht dargestellten) Steuereinrichtung geführt wird und die Einschaltung des Motors 21 ermöglicht. Sowohl die Versorgungsleitungen als auch die Signalleitungen des Schalters 57b sind mit dem Kabel 31 verbunden.

Das Tastelement 43 bildet zusammen mit dem Schalter 56 den Drucksensor 43,56; die Tastkappe 45 bildet zusammen mit dem Schalter 56 den Spritzendetektor 45,56 und die Hülse 37 bildet zusammen mit dem Schalter 56 den Ausrichtdetektor 37,56.

Fig. 4 zeigt den Zustand, in dem die Spritze 14 ordnungsgemäß in den Druckinfusionsapparat eingesetzt ist. Dabei legt sich der Flansch 12a der Kolbenstange 15 vollflächig gegen die Tastkappe 45, so daß diese unter Zusammendrückung der Feder 46 geradegestellt wird. Dabei wird über den Stift 51 die Schaltfahne 50 um ihre Achse 52 so verschwenkt, daß sich die Öffnung 50a im Strahlenweg der Lichtschranke 57 befindet. Die Schaltfahne 53 befindet sich bei eingedrücktem Betätigungsorgan 19 außerhalb des Lichtwegs der Lichtschranke 57. In diesem Zustand gelangt also das Licht der Lichtquelle 57a zum Empfänger 57b, so daß die Steuereinrichtung den Motor 21 auf Antrieb schalten kann.

Fig. 5 zeigt den Zustand, daß keine Spritze eingelegt ist. Die Tastkappe 45 wird von der Feder 46 um den Stift 47 herum in ihre Schräglage gekippt. Da der Stift 51 an dem dem Stift 47 entgegengesetzten Bereich des Umfangs der Tastkappe 45 angreift, wird die Schaltfahne 50 so verschwenkt, daß ihre Öffnung 50a aus dem Strahlenweg der Lichtschranke 57 herausgeht und die Schaltfahne den Strahlenweg unterbricht. Der Motor 21 kann in diesem Zustand nicht betrieben werden und ggf. wird ein Alarmsignal erzeugt.

Fig. 6 zeigt den Zustand, daß zur Ermöglichung des manuellen Drehens der Spindel 25 an dem Betätigungsorgan 19 gezogen wird, um die Axialverzahnung 39 außer Eingriff mit dem Gehäuse 42 zu bringen. Hierdurch wird infolge des Eingriffs des Stiftes 55 in die mit dem Betätigungsorgan 19 verbundene Hülse 37 die Schaltfahne 53 so verschwenkt, daß sie den Strahlenweg der Lichtschranke 57 blockiert. In diesem Zustand kann das Betätigungsorgan 19 gedreht werden, um die Spindel 25 zu drehen und damit den Schieber 17 zusammen mit dem Spritzenhalter 16 in Längsrichtung der Spritze 14 zu verschieben, jedoch ist dabei der Motor 21 abgeschaltet und ggf. wird ein Alarmsignal erzeugt. Durch die Schaltfahne 53 wird in dem beschriebenen Zustand in jedem Fall der Strahlenweg der Lichtschranke 57 unterbrochen, unabhängig davon, ob die Schaltfahne 50 diesen Strahlenweg blockiert oder nicht.

Fig. 7 zeigt den Zustand bei der Druckabschaltung, wenn entweder der Kolben der Spritze 14 seine vordere Endstellung erreicht hat oder der Spritzenausgang blockiert ist, so daß die Flüssigkeit in der Spritze ein weiteres Vorschieben des Kolbens unmöglich macht. Der Flansch 12a liegt voll an der Tastkappe 45 an, so daß diese sich in gerader Stellung befindet. Die Kraftübertragung von der Tastkappe 45 auf das Tastelement 43 erfolgt an den Stellen A und B. Eine an der Rückseite der Tastkappe 45 vorgesehene Hülse 45a drückt gegen die vordere Stirnseite des Bechers 48, so daß dieser in dem Gehäuse 42 zurückweicht, wenn die Kraft der Kolbenstange 15 so groß ist, daß sie die Kraft der Feder 44 übersteigt. In diesem Fall weichen Tastkappe 45 und Tastelement 43 gleichermaßen zurück, wodurch die Lichtschranke 57 über die Schaltfahne 53 bewegt und der Lichtweg blockiert wird.

Aus den vorstehenden Erläuterungen ergibt sich, daß der Schalter 56 nur dann ein für den Antrieb des Motors 21 erforderliches Signal erzeugt, wenn 1. die Spritze ordnungsgemäß eingelegt ist und der Spritzendetektor 45,56 angesprochen hat, 2. das Betätigungsorgan 19 im eingeschobenen Zustand ist und 3. der Ausrastdetektor 37,56 nicht angesprochen hat und wenn gleichzeitig die Kolbenkraft nicht so groß ist, daß der Drucksensor 43,56 anspricht.

Bei dem vorliegenden Ausführungsbeispiel werden diese drei Bedingungen am zweiten Spritzenhalter 16 überwacht und hierzu ist ein einziger Schalter 56 vorgesehen. Abweichend von diesem Ausführungsbeispiel besteht die Möglichkeit, die drei Überwachungen an dem ersten Spritzenhalter 11 vorzunehmen oder die Überwachungsfunktionen auf die beiden Spritzenhalter 11 und 16 zu verteilen, wobei verschiedene Schalter für die Überwachungsfunktionen vorgesehen sein können. Es ist auch möglich, einen Spritzendetektor und/oder einen Drucksensor an jedem der beiden Spritzenhalter vorzusehen.

## Patentansprüche

1. Druckinfusionsapparat für medizinische Anwendungen, mit
   einem Gehäuse (10), das einen ersten Spritzenhalter (11) aufweist,
   einem relativ zum Gehäuse (10) verschiebbaren angetriebenen Schieber (17), der einen zweiten Spritzenhalter (16) aufweist,
   einem Drucksensor (43,56), der in Abhängigkeit von der auf einen der Spritzenhalter einwirkenden Kraft anspricht und ein an diesem Spritzenhalter (16) abgestütztes Tastelement (43) aufweist, welches bei Überschreiten einer vorgegebenen Spritzenkraft zurückweicht und einen Schalter (56) zum Abschalten des Antriebs für den Schieber (17) betätigt,
   und einem an dem Spritzenhalter (16) vorgesehenen Spritzendetektor,
   **dadurch gekennzeichnet,**
   daß der Spritzendetektor eine federgespannte Tastkappe (45) aufweist, die die Anwesenheit eines an dem Spritzenhalter (16) angreifenden Anschlagteils (12a) einer Spritze (14) im Spritzenhalter erkennt und die von dem Tastelement (43) des Drucksensors abgestützt ist.

2. Druckinfusionsapparat nach Anspruch 1, dadurch gekennzeichnet, daß der Drucksensor in dem Spritzenhalter (16) des Schiebers (17) angeordnet ist.

3. Druckinfusionsapparat nach Anspruch 1 oder 2, dadurch gekennzeichnet, daß der Spritzendetektor (45,56) und der Drucksensor (43,56) einen gemeinsamen Schalter (56) aufweisen, der sowohl bei entlasteten Spritzendetektor als auch bei über eine Ansprechschwelle hinaus belastetem Drucksensor betätigt ist.

4. Druckinfusionsapparat, insbesondere nach einem der Ansprüche 1 bis 3, dadurch gekennzeichnet, daß ein bei Motorbetrieb eingerastetes Betätigungsorgan (19) vorgesehen ist, das nach dem Ausrasten eine manuelle Verschiebung des Schiebers (17) ermöglicht, und daß ein Ausrastdetektor (37,56) bei Ausrasten des Betätigungsorgans (19) den Antrieb (20) abschaltet.

5. Druckinfusionsapparat nach Anspruch 3 oder 4, dadurch gekennzeichnet, daß der Ausrastdetektor (37,56) denselben Schalter (56) betätigt, wie der Drucksensor (43,56) und/oder der Spritzendetektor (45,56).

6. Druckinfusionsapparat nach einem der Ansprüche 1 bis 5, dadurch gekennzeichnet, daß der Schalter (56) eine Lichtschranke (56) aufweist, in deren Strahlengang eine von dem Drucksensor und eine von dem Ausrastdetektor verstellbare Schaltfahne (50,53) hineinragen können.

7. Druckinfusionsapparat nach einem der vorhergehenden Ansprüche, dadurch gekennzeichnet, daß der Schieber (17) mit einem Führungsrohr (27) verbunden ist, in dem eine elektrische Leitung (31) entlangführt, welche von dem dem Antrieb (20) zugewandten Ende des Führungsrohres (27) über eine flexible Kabelschleife (32) in das Gehäuse (10) hineinführt.

## Claims

1. A pressure infusion device for medical applications, comprising
a housing (10) having a first syringe holder (11),
a slide (17) displaceable relative to said housing (10) and having a second syringe holder (16),
a pressure sensor (43, 56) responding to a force acting on one of said syringe holders and having a sensor element (43) supported on this syringe holder (16), which sensor element recedes when a predetermined syringe force is exceeded, and actuates a switch (56) for switching off the drive of the slide (17),
and a syringe detector provided on said syringe holder (16),
characterised in

that said syringe detector has a spring-loaded sensor cap (45) that detects the presence of a stop member (12a) of a syringe (14) within said syringe holder when said stop member engages said syringe holder (16), said cap being supported by said sensor element (43) of said pressure sensor.

2. The pressure infusion device of claim 1, characterised in that said pressure sensor is arranged in said syringe holder (16) of said slide (17).

3. The pressure infusion device of claim 1 or 2, characterised in that said syringe detector (45, 56) and said pressure sensor (43, 56) have a common switch (56) that is operated both when said syringe detector is relieved and when said pressure sensor is loaded beyond a response threshold.

4. A pressure infusion device, in particular according to one of claims 1 to 3, characterised in that an actuating member (19), engaged during the operation of the motor, is provided which allows a manual displacement of the slide (17) after disengagement, and that a disengagement detector (37, 56) cuts off the drive (20) upon disengagement of said actuating member (19).

5. The pressure infusion device of claim 3 or 4, characterised in that said disengagement detector (37, 56) actuates the same switch (56) as said pressure sensor (43, 56) and/or said syringe detector (45, 56).

6. The pressure infusion device of one of claims 1 to 5, characterised in that said switch (56) has a light barrier (56), into the beam path of which a switching lug (50, 53), displaceable by said pressure sensor and by said disengagement detector, can protrude.

7. The pressure infusion device of one of the preceding claims, characterised in that said slide (17) is connected with a guiding pipe (27) through which an electric wire (31) extends leading from the end of said guiding pipe (27) averted from said drive into said housing (10) via a flexible cable loop (32).

## Revendications

1. Appareil de perfusion sous pression à usage médical, comprenant
un boîtier (10) qui comporte un premier support de seringue (11),

un coulisseau (17) entraîné, pouvant coulisser par rapport au boîtier (10) et comportant un second support de seringue (16),

un détecteur de pression (43, 56) qui réagit en fonction de la force agissant sur l'un des supports de seringue et présente un palpeur (43) qui s'appuie sur ce support de seringue (16) et qui dans le cas d'un dépassement d'une force de seringue prédéfinie, se rétracte et actionne un commutateur (56) pour arrêter le dispositif d'entraînement du coulisseau (17),

et un détecteur de seringue prévu sur le support de seringue (16),

caractérisé

en ce que le détecteur de seringue comporte un capuchon de détection (45) qui est soumis à la tension d'un ressort, qui permet d'identifier la présence, dans le support de seringue, d'une pièce de butée (12a) d'une seringue (14) coopérant avec le support de seringue (16), et qui est maintenu par le palpeur (43) du détecteur de pression.

2. Appareil de perfusion sous pression selon la revendication 1, caractérisé en ce que le détecteur de pression est disposé dans le support de seringue (16) du coulisseau (17).

3. Appareil de perfusion sous pression selon la revendication 1 ou 2, caractérisé en ce que le détecteur de seringue (45, 56) et le détecteur de pression (43, 56) comportent un commutateur commun (56) qui est actionné, aussi bien pour un détecteur de seringue non sollicité, que pour un détecteur de pression sollicité au-delà d'un seuil de réaction.

4. Appareil de perfusion sous pression, notamment selon l'une des revendications 1 à 3, caractérisé en ce qu'est prévu un organe d'actionnement (19) qui est encliqueté lors du fonctionnement du moteur et qui permet, après décliquetage, un déplacement manuel du coulisseau (17), et en ce qu'un détecteur de décliquetage (37, 56) arrête le dispositif d'entraînement (20) lors du décliquetage de l'organe d'actionnement (19).

5. Appareil de perfusion sous pression selon la revendication 3 ou 4, caractérisé en ce que le détecteur de décliquetage (37, 56) actionne le même commutateur (56) que le détecteur de pression (43, 56) et/ou le détecteur de seringue (45, 56).

6. Appareil de perfusion sous pression selon l'une des revendications 1 à 5, caractérisé en ce que le commutateur (56) comporte une barrière photo-électrique (56) dans la trajectoire du rayon de laquelle peuvent s'engager des écrans de commande (50, 53) pouvant être déplacés respectivement, l'un par le détecteur de pression et l'autre par le détecteur de décliquetage.

7. Appareil de perfusion sous pression selon l'une des revendications précédentes, caractérisé en ce que le coulisseau (17) est relié à un tube de guidage (27) le long duquel chemine une conduite électrique (31), qui, à partir de l'extrémité du tube de guidage (27), située du côté du dispositif d'entraînement (20), conduit vers l'intérieur du boîtier (10) par l'intermédiaire d'une boucle de câble flexible (32).

FIG.1

FIG. 2

FIG. 3

FIG. 4

FIG. 5

FIG. 6

FIG. 7